# EUROPÄISCHE PATENTSCHRIFT

(11) **EP 3 836 890 B1**
(45) Veröffentlichungstag und Bekanntmachung des Hinweises auf die Patenterteilung: **27.12.2023**
(21) Anmeldenummer: 19726944.2
(22) Anmeldetag: 22.05.2019
(51) Int. Cl.: A61K 8/41, A61K 8/49, A61K 8/58, A61Q 5/06, A61K 8/86

(54) **VERFAHREN ZUM FÄRBEN VON KERATINISCHEM MATERIAL, UMFASSEND DIE ANWENDUNG VON EINER SILICIUMORGANISCHEN VERBINDUNG, EINEM TENSID UND EINEM PIGMENT**
METHOD FOR DYEING KERATINOUS MATERIAL, COMPRISING THE USE OF AN ORGANOSILICON COMPOUND, A SURFACTANT AND A PIGMENT
PROCÉDÉ POUR LA COLORATION DE MATÉRIAU KÉRATINIQUE, COMPRENANT L'UTILISATION D'UN COMPOSÉ ORGANIQUE DU SILICIUM, UN TENSIOACTIF ET UN PIGMENT

(30) Priorität: 16.08.2018 DE 102018213814
(43) Veröffentlichungstag der Anmeldung: 23.06.2021
(73) Patentinhaber: Henkel AG & Co. KGaA, 40589 Düsseldorf (DE)
(72) Erfinder: LECHNER, Torsten, 40764 Langenfeld (DE); SCHOEPGENS, Juergen, 41366 Schwalmtal (DE); NOWOTTNY, Marc, 41069 Mönchengladbach (DE); WESER, Gabriele, 41472 Neuss (DE); SCHUMACHER, Ulrike, 40589 Düsseldorf (DE); KOLONKO, Claudia, 42857 Remscheid (DE); KRIENER, Caroline, 40229 Düsseldorf (DE); MATHIASZYK, Carsten, 45277 Essen (DE)
(86) Internationale Anmeldenummer: PCT/EP2019/063202
(87) Internationale Veröffentlichungsnummer: WO 2020/035187

(56) Entgegenhaltungen:
- EP-A2- 2 168 633
- WO-A1-2018/187246
- US-A1- 2015 320 672
- US-A1- 2018 055 751

## Beschreibung

Gegenstand der vorliegenden Anmeldung ist ein Verfahren zum Färben von keratinischem Material, insbesondere menschlichen Haaren, welches die Anwendung von mindestens zwei verschiedenen Mitteln (a) und (b) umfasst. Das Mittel (a) enthält mindestens eine organische Siliciumverbindung der Formel (I), mindestens eine organische Siliciumverbindung der Formel (IV) und mindestens ein Tensid. Das Mittel (b) enthält mindestens ein Pigment.

Ein zweiter Gegenstand dieser Anmeldung ist eine Mehrkomponenten-Verpackungseinheit (Kit-of-parts) zum Färben von keratinischem Material, insbesondere menschlichen Haaren, welche getrennt voneinander konfektioniert in zwei verschiedenen Containern die Mittel (a) und (b) umfasst.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Shampoonierungen besonders widerstandsfähig sind. Als organischer Silicium-Verbindung wurde beispielsweise 3-Aminopropyltriethoxysilan eingesetzt.

In WO 2018/115059 A1 wird ein Färbeverfahren beschrieben, welches in mehreren Schritten abläuft. Ein Schritt umfasst die Anwendung eines direktziehendenden Farbstoffs, und in einem weiteren Schritt wird ein Organosilan auf die Haare appliziert. Auch mit diesem Verfahren sollen Färbungen mit guten Waschechtheiten erzielt werden. Die in dieser Schrift verwendeten Silane sind beispielsweise 3-Aminopropyltriethxoysilan und Methyltrimethoxysilan.

In den Färbeverfahren von EP 2168633 B1 und WO 2018/115059 A1 werden siliciumorganische Verbindungen aus der Gruppe der Silane eingesetzt, wobei die Molekülstruktur dieser Silane mindestens eine Hydroxygruppe und/oder hydrolysierbare Gruppe umfasst. Aufgrund der Anwesenheit der Hydroxygruppen bzw. hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung bzw. Polymerisierung der Silane führt bei Anwendung auf dem Keratinmaterial letztendlich zur Ausbildung eines Films, welcher die farbgebenden Verbindungen fixiert und auf diese Weise sehr langlebige Färbungen erzeugt.

Bei näherer Untersuchung der in EP 2168633 B1 und WO 2018/115059 A1 offenbarten Färbeverfahren hat sich gezeigt, dass die in diesen Färbeverfahren erhaltenen Farbresultate immer noch der Verbesserung bedürfen. Insbesondere wird nach wie vor nach Methoden gesucht, die Farbintensität von Färbungen, die durch Einsatz von Pigmenten erhalten wurden, noch weiter zu verbessern. Zudem ist es eine bislang ungelöste Aufgabe, mit dem Einsatz von Pigmenten eine aufhellende Wirkung zu erzielen.

Es war die Aufgabe der vorliegenden Erfindung, ein Färbesystem bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie beispielsweise Pigmente) in extrem dauerhafter Weise auf den Haaren zu fixieren. Hierbei sollten besonders gleichmäßige Färbungen mit hoher Farbintensität erhalten werden. Darüber hinaus sollte mit Anwendung des Verfahrens ein Farbresultat erzielt werden, welches auch heller als die Ausgangshaarfarbe sein kann. Mit anderen Worten sollte es möglich sein, die Haare unter Einsatz von Pigmenten aufzuhellen und auf diese Weise den Anschein einer natürlichen Blondierung zu ermöglichen.

Überraschenderweise hat sich nun herausgestellt, dass die vorgenannte Aufgabe hervorragend gelöst werden kann, wenn keratinische Materialien, insbesondere Haare, mit einem Verfahren gefärbt werden, bei welchem mindestens zwei Mittel (a) und (b) auf die keratinischen Materialien (Haare) appliziert werden. Hierbei enthält das Mittel (a) mindestens eine organische Siliciumverbindung und darüber hinaus mindestens ein Tensid. Das Mittel (b) beinhaltet mindestens ein Pigment. Bei Einsatz der beiden Mittel (a) und (b) in einem Färbeverfahren wurden Färbungen mit besonders hoher Farbintensität und großer Gleichmäßigkeit erhalten. Das Egalisiervermögen dieser Färbungen war hervorragend.

Bei Färbung von dunklen Haarsträhnen mit dem erfindungsgemäßen Verfahren konnte - sofern im Mittel (b) helle Pigmente eingesetzt wurden - auch eine Hellerfärbung der Haarsträhnen erzielt werden. Aufgrund der großen Gleichmäßigkeit des Farbergebnisses sahen die auf diesem Wege erhaltenen Blondfärbungen sehr natürlich aus.

Ein erster Gegenstand der vorliegenden Erfindung ist daher ein Verfahren zum Färben von keratinischem Material, insbesondere menschlichen Haaren, gemäß Anspruch 1. Dieses Verfahren umfasst die Anwendung der Mittel (a) und (b), wobei das Mittel (a) mindestens eine organische Siliciumverbindung der Formel (I) und mindestens eine organische Siliciumverbindung der Formel (IV) sowie mindestens ein Tensid enthält, und das Mittel (b) mindestens ein Pigment enthält.

### keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a) und (b)

Im Rahmen des erfindungsgemäßen Verfahrens werden die Mittel (a) und (b) auf dem keratinischen Material, insbesondere den menschlichen Haaren, appliziert. Die beiden Mittel (a) und (b) sind voneinander verschieden.

### Mittel (a)

Als ersten erfindungwesentlichen Inhaltsstoff enthält das Mittel (a) mindestens eine organische Siliciumverbindung der Formel (I) und mindestens eine organische Siliciumverbindung der Formel (IV).

Diese im Mittel (a) enthaltenen organischen Siliciumverbindungen bzw. organischen Silane sind reaktive Verbindungen.

Das Mittel (a) enthält die organischen Siliciumverbindungen in einem kosmetischen Träger, der wasserhaltig, wasserarm oder auch wasserfrei sein kann. Zudem kann der kosmetische Träger flüssig, gelartig, cremeförmig, pulverförmig oder auch fest (z.B. in Form einer Tablette oder eines Presslings) sein. Bevorzugt ist der kosmetische Träger des Mittels (a) ein wässriger oder wässrig-alkoholischer Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Der kosmetische Träger ist bevorzugt wasserhaltig, was bedeutet, dass der Träger - bezogen auf sein Gewicht - mindestens 2 Gew.-% Wasser enthält. Bevorzugt liegt der Wassergehalt oberhalb von 5 Gew.-%, weiter bevorzugt oberhalb von 10 Gew.-% noch weiter bevorzugt oberhalb von 15 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die Pigmente in einem besonders homogenen, gleichmäßigen und glatten Film an der Oberfläche des Keratinmaterials ab. Der Film bildet sich in situ durch Oligomerisierung bzw. Polymerisierung des oder der organischen Siliciumverbindungen, sowie durch die Wechselwirkung von organischer Silicumverbindung mit dem oder den Pigmenten.

### Organische Siliciumverbindungen

Als ersten erfindungswesentlichen Bestandteil enthält das Mittel (a) mindestens eine organische Siliciumverbindung der Formel (I) und mindestens eine organische Siliciumverbindung der Formel (IV).

Das Mittel (a) enthält eine organische Siliciumverbindung der Formel (I)

R₁R₂N-L-Si(OR₃)a(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Silicumverbindungen der Formel (I) sind
- (3-Aminopropyl)triethoxysilan 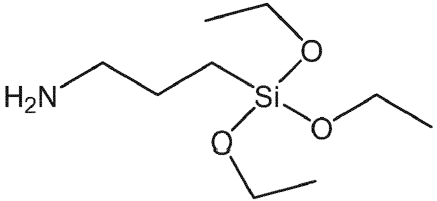
- (3-Aminopropyl)trimethoxysilan 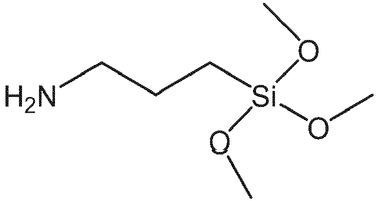
- (2-Aminoethyl)triethoxysilan 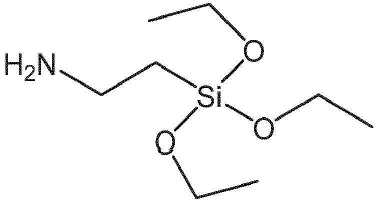 und
- (2-Aminoethyl)trimethoxysilan

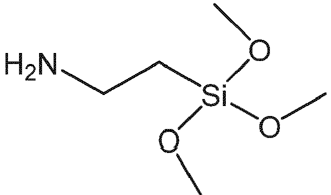

In einer bevorzugten Ausführungsform ist ein erfindungsgemäßes Verfahren daher dadurch gekennzeichnet, dass das Mittel (a) mindestens eine organische Siliciumverbindung enthält, die ausgewählt ist aus der Gruppe aus
- (3-Aminopropyl)triethoxysilan
- (3-Aminopropyl)trimethoxysilan
- (2-Aminoethyl)triethoxysilan und
- (2-Aminoethyl)trimethoxysilan.

Die vorgenannten organische Siliciumverbindung der Formel (I) sind kommerziell erhältlich.

(3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Das im erfindungsgemäßen Verfahren auf dem keratinischen Material angewendete Mittel (a) enthält mindestens eine organische Siliciumverbindung der Formel (IV)

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkyl-alkoxy-silane oder der Alkyl-hydroxy-silane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe. Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgrurppe oder eine n-Octylgruppe.

In den organischen Siliciumverbindungen der Forml (IV) steht der Rest R₁₀ für ein Wasserstoffatom oder eine C₁-C₆-Alkylgruppe. Besonders bevorzugt steht R10 für eine Methylgruppe oder für eine Ethylgruppe.

In den organischen Siliciumverbindungen der Forml (IV) steht der Rest R₁₁ für eine C₁-C₆-Alkylgruppe. Besonders bevorzugt steht R11 für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn im Verfahren ein Mittel (a) eingesetzt wurde, welches mindestens eine organische Siliciumverbindung der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Silicumverbindungen der Formel (IV) sind
- Methyltrimethoxysilan 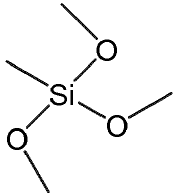
- Methyltriethoxysilan 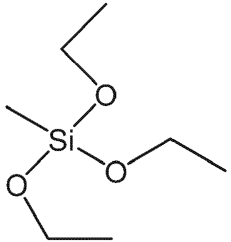
- Ethyltrimethoxysilan 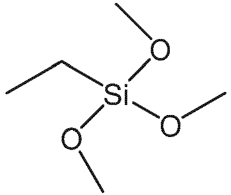
- Ethyltriethoxysilan 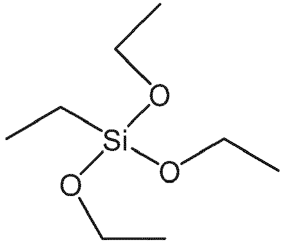
- n-Hexyltrimethoxysilan 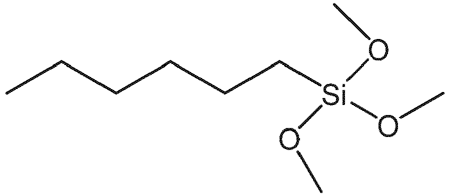
- n-Hexyltriethoxysilan 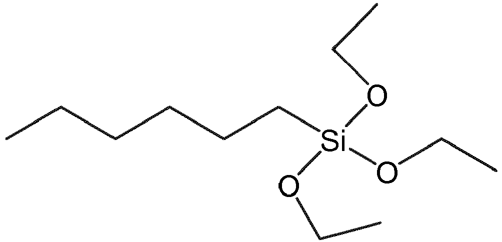
- n-Octyltrimethoxysilan 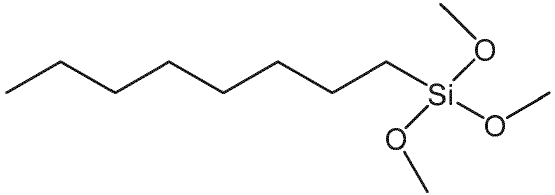
- n-Octyltriethoxysilan 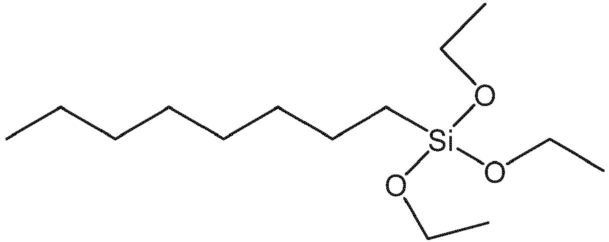
- n-Dodecyltrimethoxysilan und/oder 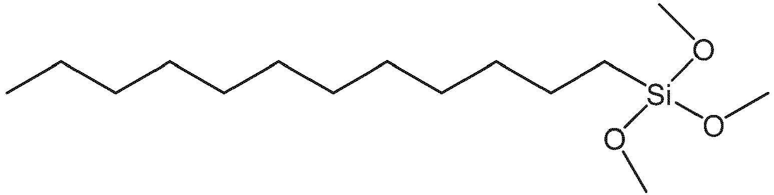
- n-Dodecyltriethoxysilan.

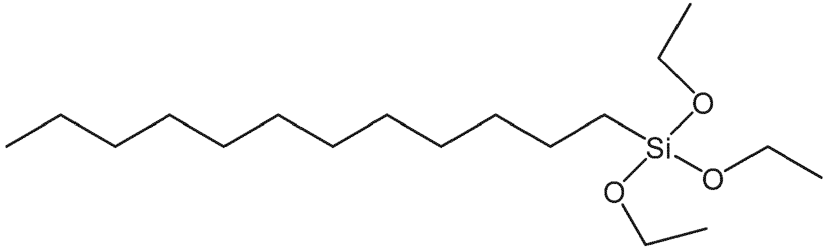

In einer weiteren bevorzugten Ausführungsform ist ein erfindungsgemäßes Verfahren dadurch gekennzeichnet, dass das Mittel (a) mindestens eine organische Siliciumverbindung der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan und/oder
- Dodecyltriethoxysilan.

Bei den zuvor beschriebenen organischen Siliciumverbindungen handelt es sich um reaktive Verbindungen. In diesem Zusammenhang hat es sich als bevorzugt herausgestellt, wenn das erfindungsgemäße Mittel (a) - bezogen auf das Gesamtgewicht des Mittels (a) - eine oder mehrere organische Siliciumverbindungen in einer Gesamtmenge von 0,1 bis 20,0 Gew.-%, bevorzugt 1,0 bis 15,0 Gew.-% und besonders bevorzugt 2,0 bis 8,0 Gew.-% enthält.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen in einer Gesamtmenge von 0,1 bis 20,0 Gew.-%, bevorzugt 1,0 bis 15,0 Gew.-% und besonders bevorzugt 2,0 bis 8,0 Gew.-% enthält.

Zur Erzielung von besonders guten Färbeergebnissen ist es insbesondere von Vorteil, die organische Siliciumverbindungen der Formel (I) und/oder (II) in bestimmten Mengenbereichen im Mittel (a) einzusetzen. Besonders bevorzugt enthält das Mittel (a) - bezogen auf das Gesamtgewicht des Mittels (a) - ein oder mehrere organische Siliciumverbindungen der Formel (I) in einer Gesamtmenge von 0,1 bis 10,0 Gew.-%, bevorzugt 0,5 bis 5,0 Gew.-% und besonders bevorzugt 0,5 bis 3,0 Gew.-%.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen der Formel (I) in einer Gesamtmenge von 0,1 bis 10,0 Gew.-%, bevorzugt 0,5 bis 5,0 Gew.-% und besonders bevorzugt 0,5 bis 3,0 Gew.-% enthält.

Weiterhin hat es sich als ganz besonders bevorzugt herausgestellt, wenn auch das oder die organische Siliciumverbindungen der Formel (IV) in bestimmten Mengenbereichen im Mittel (a) enthalten sind. Besonders bevorzugt enthält das Mittel (a) - bezogen auf das Gesamtgewicht des Mittels (a) - eine oder mehrere organische Siliciumverbindungen der Formel (IV) in einer Gesamtmenge von 0,1 bis 20,0 Gew.-%, bevorzugt 2,0 bis 15,0 Gew.-% und besonders bevorzugt 4,0 bis 9,0 Gew.-%.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen der Formel (IV) in einer Gesamtmenge von 0,1 bis 20,0 Gew.-%, bevorzugt 2,0 bis 15,0 Gew.-% und besonders bevorzugt 3,2 bis 10,0 Gew.-% enthält.

Im Zuge der zu dieser Erfindung führenden Arbeiten hat sich herausgestellt, dass auf dem Keratinmaterial besonders stabile und gleichmäßige Filme erhalten werden konnten, wenn das Mittel (a) zwei strukturell voneinander verschiedene organische Siliciumverbindungen enthält.

In einer explizit ganz besonders bevorzugten Ausführungsform ist ein erfindungsgemäßes Verfahren dadurch gekennzeichnet, dass auf dem keratinischen Material ein Mittel (a) angewendet wird, welches mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe aus Methyltrimethoxysilan, Methyltriethoxysilan, Ethyltrimethoxysilan und Ethyltriethoxysilan ausgewählt ist.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - enthält:
- 0,5 bis 3,0 Gew.-% mindestens einer ersten organischen Silicumverbindung, die ausgewählt ist aus der Gruppe aus (3-Aminopropyl)trimethoxysilan, (3-Aminopropyl)triethoxysilan, (2-Aminoethyl)trimethoxysilan und (2-Aminoethyl)triethoxysilan, und
- 3,2 bis 10,0 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Octyltrimethoxysilan, Octyltriethoxysilan, Dodecyltrimethoxysilan und Dodecyltriethoxysilan.

Im Rahmen dieser Ausführungsform enthält das Mittel (a) eine oder mehrere organischen Silicumverbindungen einer ersten Gruppe in einer Gesamtmenge von 0,5 bis 3,0 Gew.-%. Die organischen Silicumverbindungen dieser ersten Gruppe sind ausgewählt aus der Gruppe aus (3-Aminopropyl)trimethoxysilan, (3-Aminopropyl)triethoxysilan, (2-Aminoethyl)trimethoxysilan und (2-Aminoethyl)triethoxysilan.

Im Rahmen dieser Ausführungsform enthält das Mittel (a) eine oder mehrere organischen Silicumverbindungen einer zweiten Gruppe in einer Gesamtmenge von 3,2 bis 10,0 Gew.-%. Die organischen Silicumverbindungen dieser zweiten Gruppe sind ausgewählt aus der Gruppe aus Methyltrimethoxysilan, Methyltriethoxysilan, Ethyltrimethoxysilan, Ethyltriethoxysilan, Octyltrimethoxysilan, Octyltriethoxysilan, Dodecyltrimethoxysilan und/oder Dodecyltriethoxysilan.

### Tenside

Als zweiten wesentlichen Bestandteil enthält das im erfindungsgemäßen Verfahren eingesetzte Mittel (a) mindestens ein Tensid. Als ganz besonders bevorzugt hat sich der Einsatz von kationischen und/oder nichtionischen Tensiden herausgestellt.

In einer weiteren bevorzugten Ausführungsform ist ein erfindungsgemäßes Verfahren dadurch gekennzeichnet, dass das Mittel (a) mindestens ein Tensid aus der Gruppe der kationischen und/oder nichtionischen Tenside enthält.

Im Rahmen einer Ausführungsform enthält das im erfindungsgemäßen Verfahren eingesetzte Mittel (a) mindestens ein kationisches Tensid.

Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren and Grenzflächen und aggregieren in wässriger Lösung oberhalb der kritischen Micellbildungskonzentration zu positiv geladenen Micellen.

Beispiele für Kationtenside sind
- quartäre Ammniumverbindungen, die als hydrophobe Reste ein oder zwei Alkylketten mit einer Kettenlänge von 8 bis 28 C-Atomen tragen können
- quartäre Phosphoniumsalze, substituert mit einer oder mehrern Alkylketten mit einer einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, diese ist beispielsweise bei Esterquats der Fall.

Besonders gleichmäßige Filme konnten erhalten werden, wenn im erfindungsgemäßen Verfahren ein Mittel (a) eingesetzt wurde, welches mindestens ein kationisches Tensid der Formel (X) enthält, 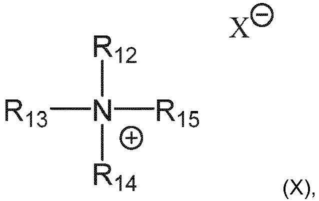 worin
- R12, R13, R14: unabhängig voneinander für eine C₁-C₆-Alkylgruppe, eine C₂-C₆-Alkenylgruppe oder eine C₂-C₆-Hydroxyalkylgruppe stehen,
- R15: für eine C₈-C₂₈-Alkylgruppe steht und
- X-: für ein physiologisch verträgliches Anion steht.

In einer weiteren bevorzugten Ausführungsform ist ein erfindungsgemäßes Verfahren dadurch gekennzeichnet, dass das Mittel (a) mindestens ein kationisches Tensid der Formel (X) enthält, 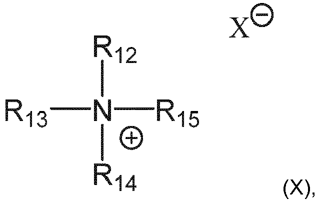 worin
- R12, R13, R14: unabhängig voneinander für eine C₁-C₆-Alkylgruppe, eine C₂-C₆-Alkenylgruppe oder eine C₂-C₆-Hydroxyalkylgruppe stehen,
- R15: für eine C₈-C₂₈-Alkylgruppe steht und
- X-: für ein physiologisch verträgliches Anion steht.

Die Reste R12, R13 und R14 stehen unabhängig voneinander für eine C₁-C₆-Alkylgruppe, eine C₂-C₆-Alkenylgruppe oder eine C₂-C₆-Hydroxyalkylgruppe.

Bevorzugt stehen R12, R13 und R14 unabhängig voneinander für eine Methylgruppe, für eine Ethylgruppe oder für eine 2-Hydroxyethylgruppe. Ganz besonders bevorzugt stehen R12, R13 und R14 für eine Methylgruppe.

Der Rest R15 steht für eine C₈-C₂₈-Alkylgruppe. Bevorzugt steht R15 für eine C₁₂-Alkylgruppe, eine C₁₄-Alkylgruppe, eine C₁₆-Alkyglruppe, eine C₁₈-Alkyglruppe, eine C₂₀-Alkylgruppe oder für eine C₂₂-Alkylgruppe. Die vorgenannten Alkylgruppen sind bevorzugt linear. Ganz besonders bevorzugt steht R15 für eine lineare C₁₆-Alkyglruppe, eine lineare C₁₈-Alkyglruppe oder eine lineare C₂₀-Alkyglruppe.

Ein besonders bevorzugtes kationisches Tensid der Formel (X) ist beispielsweise Cetrimonium Chlorid (Trimethyl hexadecyl ammonium chloride), das unter dem Handelsnamen Genamin CTAC von Clariant, oder unter dem Handelsnamen Dehyquart A-CA von der BASF bezogen werden kann.

Weiterhin ist es auch besonders bevorzugt, wenn das im erfindungsgemäßen Verfahren eingesetzte Mittel (a) als kationisches Tensid mindestens eine Imidazoliumverbindung enthält.

Innerhalb der Gruppe der kationischen Imidazoliumverbindungen haben sich Verbindungen der Formel (XI) als besonders gut kompatibel mit den organischen Siliciumverbindungen herausgestellt 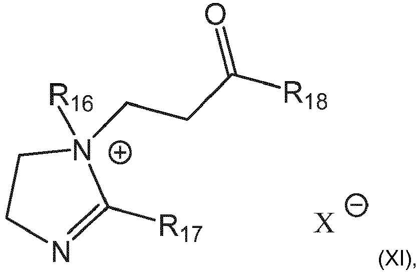 worin
- R16: für eine C₁-C₆-Alkylgruppe steht
- R17, R18: unabhängig voneinander für eine C₇-C₂₇-Alkylgruppe stehen und
- X-: für ein physiologisch verträgliches Anion steht.

Bei Einsatz der kationischen Imidazoliumverbindungen (XI) im Mittel (a) konnten zudem sehr intensive und gleichmäßige Färbungen erhalten werden.

In einer weiteren bevorzugten Ausführungsform ist ein erfindungsgemäßes Verfahren dadurch gekennzeichnet, dass das Mittel (a) mindestens ein kationisches Tensid der Formel (XI) enthält, 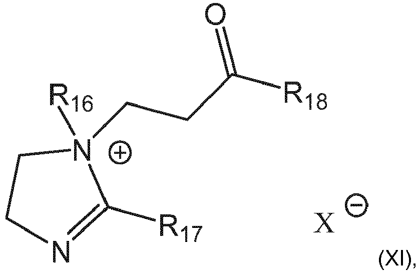 worin
- R16: für eine C₁-C₆-Alkylgruppe steht
- R17, R18: unabhängig voneinander für eine C₇-C₂₇-Alkylgruppe stehen und
- X-: für ein physiologisch verträgliches Anion steht.

Keratinische Fasern konnten insbesondere dann in intensiven Nuancen gefärbt werden, wenn in den erfindungsgemäßen Mitteln (a) neben den organischen Siliciumverbindungen mindestens ein kationisches Tensid der Formel (XI) eingesetzt wurde, bei welchem
- R16: für eine Methylgruppe steht,
- R17, R18: unabhängig voneinander für eine C₈-C₂₇-Alkylgruppe stehen und
- X-: für ein physiologisch verträgliches Anion steht.

Besonders bevorzugte Verbindungen der Formel (XI) sind unter dem INCI-Namen Quaternium-87 bekannt, die auch unter den Handelsnamen Varisoft W 575 PB oder Revoquat PG 75 vertrieben werden.

Ebenfalls besonders intensive Färbeergebnisse konnten erzielt werden, wenn als kationische Tenside eine oder mehrere Verbindungen aus der Gruppe der Esterquats im Mittel (a) eingesetzt wurden. Der Name Esterquats ist eine Sammelbezeichnung für kationische grenzflächenaktive Verbindungen mit zwei hydrophoben Gruppen, die über Ester-Bindungen mit einem quaternisierten Di- bzw. Triethanolamin oder einer analogen Verbindung verknüpft sind.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Ganz besonders gut geeignete Esterquats sind die Verbindungen der Formel (XII), 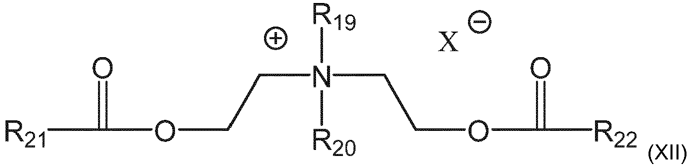 worin
- R19, R20: unabhängig voneinander für eine C₁-C₆-Alkylgruppe oder eine C₂-C₆-Hydroxyalkylgruppe stehen,
- R21, R22: unabhängig voneinander für eine C₇-C₂₇-Alkylgruppe stehen und
- X-: für ein physiologisch verträgliches Anion steht.

In einer weiteren bevorzugten Ausführungsform ist ein erfindungsgemäßes Verfahren dadurch gekennzeichnet, dass das Mittel (a) mindestens ein kationisches Tensid de Formel (XII) enthält, 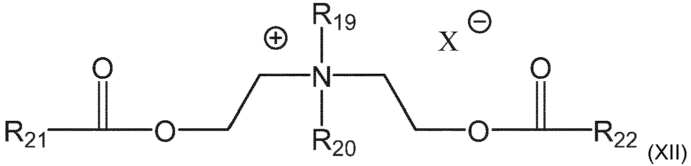 worin
- R19, R20: unabhängig voneinander für eine C₁-C₆-Alkylgruppe oder eine C₂-C₆-Hydroxyalkylgruppe stehen,
- R21, R22: unabhängig voneinander für eine C₇-C₂₇-Alkylgruppe stehen und
- X-: für ein physiologisch verträgliches Anion steht.

In einer weiteren besonders bevorzugten Ausführungsform ist ein erfindungsgemäßes Verfahren dadurch gekennzeichnet, dass das Mittel (a) eine oder mehrere Verbindungen der Formel (XII) enthält, 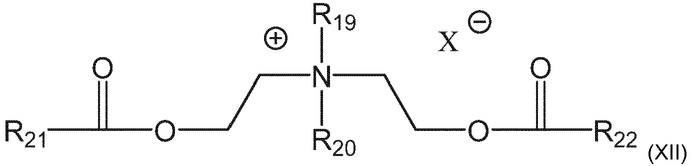 worin
- R19, R20: unabhängig voneinander für eine Methylgruppe, eine Ethylgruppe, eine Propylgruppe oder eine 2-Hydroxyethylgruppe stehen,
- R21, R22: unabhängig voneinander für eine C15-Alkylgruppe oder eine C17-Alkylgruppe stehen und
- X⁻: für ein physiologisch verträgliches Anion steht.

X⁻ steht in den Formeln (X) bis (XII) jeweils für ein physiologisch verträgliches Anion. Geeignete physiologisch verträgliche Anionen sind Halogenid (insbesondere Chlorid oder Bromid), Hydrogensulfat, Sulfat, Benzolsulfonat, p-Toluolsulfonat, Acetat, Citrat, Lactat, Tartrat, Methylsulfat (H₃COSO₃⁻), Methylsulfoant oder Trifluormethansulfonat. Besonders bevorzugt steht A⁻ für Chlorid, Bromid oder für Methylsulfat (H₃COSO₃⁻).

Das im erfindungsgemäßen Verfahren eingesetzte Mittel (a) enthält das bzw. die kationischen Tenside bevorzugt innerhalb bestimmter Mengenbereiche.

In einer weiteren besonders bevorzugten Ausführungsform ist ein erfindungsgemäßes Verfahren daher dadurch gekennzeichnet, dass das Mittel (a) - bezogen auf das Gesamtgewicht des Mittels (a) - ein oder mehrere kationische Tenside (b) in einer Gesamtmenge von 0,1 bis 10,0 Gew.-%, bevorzugt von 0,2 bis 8,0 Gew.-%, weiter bevorzugt von 0,3 bis 6,0 Gew.-% und besonders bevorzugt von 0,4 bis 4,0 Gew.-% enthält.

Im Rahmen einer weiteren Ausführungsform enthält das im erfindungsgemäßen Verfahren eingesetzte Mittel (a) mindestens ein nichtionisches Tensid.

Nichtionische Tenside sind Tenside, welche keine Ladungen, sondern starke Dipolmomente aufweisen und in wässriger Lösung stark hydratisiert sind.

Besonders gut geeignete nichtionische Tenside enthalten als hydrophile Gruppe z.B. eine Polyol-Gruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (Tnio-3),

   R⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

      R⁷CO-(NR⁸) -CH₂ - [CH(OH)]₄ - CH₂OH (Tnio-4)
   Vorzugsweise werden als Fettsäure-N-alkylpolyhydroxyalkylamide Glucamide der Formel (Tnio-4) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Das im erfindungsgemäßen Verfahren eingesetzte Mittel (a) enthält das bzw. die nichtionischen Tenside bevorzugt innerhalb bestimmter Mengenbereiche.

In einer weiteren besonders bevorzugten Ausführungsform ist ein erfindungsgemäßes Verfahren daher dadurch gekennzeichnet, dass das Mittel (a) - bezogen auf das Gesamtgewicht des Mittels (a) - ein oder mehrere nichtionische Tenside in einer Gesamtmenge von 0,1 bis 10,0 Gew.-%, bevorzugt von 0,2 bis 8,0 Gew.-%, weiter bevorzugt von 0,3 bis 6,0 Gew.-% und besonders bevorzugt von 0,4 bis 4,0 Gew.-% enthält.

### Mittel (b)

Das Mittel (b) ist gekennzeichnet durch seinen Gehalt an mindestens einem Pigment. Das Mittel (b) kann auch als Färbemittel (b) bezeichnet werden.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel (b) dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Mittel (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(lron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, C! 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)
Weiterhin besonders bevorzugte Farbpigmente mit der Handelsbezeichnung Xirona^{®} sind beispielsweise:
   Xirona Golden Sky, Merck, Silica, CI 77891 (Titanium Dioxide), Tin Oxide
   Xirona Caribbean Blue, Merck, Mica, CI 77891 (Titanium Dioxide), Silica, Tin Oxide
   Xirona Kiwi Rose, Merck, Silica, CI 77891 (Titanium Dioxide), Tin Oxide
   Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (b) ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Mittel (b) mindestens eine farbgebende Verbindungen aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (b) des erfindungsgemäßen Verfahrens ganz besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des Mittels (b), eingesetzt werden.

### filmbildendes, hydrophobes Polymer

Zur Erzielung von gleichmäßigen und besonders waschechten Färbungen hat es sich weiterhin als ganz besonders bevorzugt herausgestellt, wenn das im erfindungsgemäßen Verfahren eingesetzte Mittel (b) zusätzlich mindestens ein filmbildendes, hydrophobes Polymer enthält.

In einer weiteren bevorzugten Ausführungsform ist ein erfindungsgemäßes Verfahren dadurch gekennzeichnet, dass das Mittel (b) mindestens ein filmbildendes, hydrophobes Polymer enthält.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Unter einem filmbildenden Polymer wird im Sinne der Erfindung ein Polymer verstanden, welches in der Lage ist, auf einem Substat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein erfindungsgemäßes Verfahren dadurch gekennzeichnet, dass das Mittel (b) mindestens ein filmbildendes, hydrophobes Polymer enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C2-C10-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Me-thacrylate Copolymer), Aculyn^{®}28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®}(Acrylates/Aminoacrylates C10-30 Alkyl PEG-20 Itaconate Copolymer), Carbopol^{®} 1342, 1382, Ultrez 20, Ultrez 21 (Acrylates/C 10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Überraschenderweise hat sich herausgestellt, dass dann ganz besonders gute Färbungen mit den anionischen direktziehenden Farbstoffen erhalten werden können, wenn auch das filmbildende hydrophobe Polymere anionische Ladungen trägt.

In einer weiteren explizit ganz besonders bevorzugten Ausführungsform ist ein erfindungsgemäßes Verfahren dadurch gekennzeichnet, dass das Mittel (b) mindestens ein anionisches, filmbildendes, hydrophobes Polymer enthält.

Unter einem anionischen Polymer wird ein Polymer verstanden, das Wiederholungseinheiten mit mindestens einer Carbonsäure-Gruppe, einer Sulfonsäure-Gruppe und/oder deren physiologisch verträglichen Salzen umfasst. Mit anderen Worten wird ein anionisches Polymer aus Monomeren hergestellt, die mindestens eine Carbonsäure-Gruppe, eine Sulfonsäure-Gruppe besitzen. In diesem Zusammenhang ganz besonders bevorzugt sind die zuvor genannten hydrophoben, filmbildenden Copolymere der Acrylsäure und die Copolymere der Methacrylsäure. In den Polymeren dieser Gruppe sind die Carbonsäure-Gruppen, die Sulfonsäure-Gruppen bzw. deren Salze in einer Menge enthalten, bei der gewährleistet bleibt, dass der hydrophobe Charakter des gesamten Polymers erhalten bleibt.

Das oder die erfindungsgemäßen filmbildenden hydrophoben Polymere werden bevorzugt in bestimmten Mengenbereichen im Mittel (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere Polymere in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

In einer weiteren bevorzugten Ausführungsform ist ein erfindungsgemäßes Verfahren dadurch gekennzeichnet, dass das Mittel (b) - bezogen auf das Gesamtgewicht des Mittels (b) - ein oder mehrere filmbildende hydrophobe Polymere in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Die Mittel (insbesondere das Mittel (b)) können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SOsH-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden. Die nichtionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Zur Einstellung des gewünschten pH-Wertes können die Mittel (a) und (b) auch mindestens ein Alkalisierungsmittel und/oder Acidifizierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel (a), (b) und (c) beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem C₂-C₆-Alkylgrundkörper, der mindestens eine Hydroxylgruppe trägt.

Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Dem Fachmann geläufige Acidifizierungsmittel sind beipsielsewiese Genuss-Säuren, wie beispielsweise Zitronensäure, Essigsäure, Äpfelsäure oder Weinsäure, sowie verdünnte Mineralsäuren, wie beispielsweise Salzsäure, Schwefelsäure oder Phosphorsäure.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von Keratinmaterialien

Im Rahmen des erfindungsgemäßen Verfahrens werden die Mittel (a) und (b) auf die keratinischen Materialien, insbesondere auf die menschlichen Haare, appliziert. Damit sind die Mittel (a) und (b) die anwendungsbereiten Mittel. Die Mittel (a) und (b) sind voneinander verschieden.

Die Mittel (a) und (b) können prinzipiell gleichzeitig oder sukzessive angewendet werden, wobei die sukzessive Anwendung bevorzugt ist.

Die besten Ergebnisse konnten erhalten werden, wenn das Mittel (a) als Vorbehandlungsmittel auf die Keratinmaterialien gegeben wurde und danach das Mittel (b) als Färbemittel angewendet wurde.

Ganz besonders bevorzugt ist daher ein Verfahren zum Färben von keratinischem Material, insbesondere menschlichen Haaren, umfassend die folgenden Schritte in der angegebenen Reihenfolge:
- in einem ersten Schritt Anwendung eines Mittels (a) auf dem keratinischem Material, wobei das Mittel (a) mindestens eine organische Siliciumverbindung und mindestens ein Tensid enthält, und
- in einem zweiten Schritt Anwendung eines Mittels (b) auf dem keratinischen Material, wobei das Mittel (b) mindestens ein Pigment enthält,

Die Mittel (a),und (b) werden besonders bevorzugt innerhalb ein und desselben Färbeverfahrens angewendet, was bedeutet, dass zwischen der Anwendung der Mittel (a) und (b) ein Zeitraum von maximal einigen Stunden, liegt. Ganz besonders bevorzugt liegt zwischen der Anwendung der Mittel (a) und (b) ein Zeitraum von maximal 30 Minuten.

Im Rahmen einer weiteren bevorzugten Ausführungsform ist ein erfindungsgemäßes Verfahren dadurch gekennzeichnet, dass zunächst das Mittel (a) angewendet wird, danach das Mittel (b) angewendet wird, wobei der Zeitraum zwischen der Anwendung der Mittel (a) und (b) bei maximal 24 Stunden, bevorzugt bei maximal 12 Stunden, weiter bevorzugt bei maximal 6 Stunden und ganz besonders bevorzugt bei maximal 30 Minuten liegt.

Im Rahmen des erfindungsgemäßen Verfahrens werden die Keratinmaterialien, insbesondere die menschlichen Haare, zunächst mit Mittel (a) behandelt. Im Anschluss daran wird das eigentliche Färbemittel (b) - welches die farbgebenden Verbindungen enthält - auf die Keratinmaterialien gegeben.

Bevorzugt enthält das Mittel (a) selbst keine Farbstoffe bzw. keine farbgebenden Verbindungen. Kennzeichnend für das Vorbehandlungsmittel (a) ist sein Gehalt an mindestens einer reaktiven organische Siliciumverbindung und mindestens einem Tensid. Das oder die reaktiven organischen Siliciumverbindungen (a) funktionalisieren die Haaroberfläche, sobald sie mit dieser in Kontakt kommen. Auf diesem Wege wird ein erster, noch ungefärbter Film ausgebildet. Es hat sich herausgestellt, dass dieser Film durch die Anwesenheit der Tenside im Mittel (a) besonders gleichmäßig ist, was zum einen zu intensiven Färbungen und zum anderen auch zu einem ganz besonders gleichmäßigen Farbresultat führt. Im zweiten Schritt des Verfahrens wird nun ein Färbemittel (b) auf die Haare aufgetragen. Während der Anwendung des Färbemittels (b) gehen die farbgebenden Pigmente eine Wechselwirkung mit dem Silan-Film ein und werden auf diese Weise an die Keratinmaterialien gebunden. Hierbei können die anwendungstechnischen Eigenschaften der resultierenden Färbung durch Wahl der optimalen Verfahrensbedingungen noch weiter verbessert werden.

Im Rahmen einerweiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (a) auf die Keratinmaterialien, insbesondere die menschlichen Haare, appliziert.

Nach dem Auftragen wird das Mittel (a) auf die Keratinmaterialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des erfindungsgemäßen Verfahrens kann das Mittel (a) nun von den Keratinmaterialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die Keratinmaterialien appliziert wurde, die noch mit dem Mittel (a) beaufschlagt waren.

In Schritt (4) wird nun das Mittel (b) auf die Keratinmaterialien appliziert. Nach dem Auftragen wird nun das Mittel (b) auf die Haare einwirken gelassen.

Das erfindungsgemäße Verfahren erlaubt selbst bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (6) wird nun das Mittel (b) (sowie ggf. noch vorhandenes Mittel (a)) mit Wasser aus dem Keratinmaterial ausgespült.

Im Rahmen einerweiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

In Rahmen dieser Ausführungsform erfolgt die Abfolge der Schritte (1) bis (6) bevorzugt innerhalb von 24 Stunden, besonders bevorzugt innerhalb von 6 Stunden.

### Mehrkomponenten-Verpackungseinheit (Kit-of-parts)

Im Rahmen des erfindungsgemäßen Verfahrens werden die Mittel (a) und (b) auf den Keratinmaterialien angewendet, d.h. bei den beiden Mitteln (a) und (b) handelt es sich jeweils um die anwendungsbereiten Mittel.

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung und mindestens ein Tensid enthält, wie sie bei Beschreibung des ersten Erfindungsgegenstands im Detail offenbart wurden, und
- einen zweiten Container mit einem Mittel (b), wobei das Mittel (b) mindestens ein Pigment enthält, wie es bei Beschreibung des ersten Erfindungsgegenstands im Detail offenbart wurde.

Die im Mittel (a) des Kits enthaltenen organische Siliciumverbindungen entsprechen den organischen Siliciumverbindungen, die auch im Mittel (a) des zuvor beschriebenen Verfahrens eingesetzt wurden.

Die im Mittel (a) des Kits enthaltenen Tenside entsprechen den Tensiden, die auch im Mittel (a) des zuvor beschriebenen Verfahrens eingesetzt wurden

Die im Mittel (b) des Kits enthaltenen Pigmente entsprechen den Pigmenten, die auch im Mittel (b) des zuvor beschriebenen Verfahrens eingesetzt wurden.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung und mindestens ein Tensid enthält, wobei die organische Siliciumverbindung und das Tensid bei Beschreibung des ersten Erfindungsgegenstands im Detail offenbart wurden, und
- einen zweiten Container mit einem Mittel (b), wobei das Mittel (b) mindestens ein Pigment enthält, wobei das Pigment bei Beschreibung des ersten Erfindungsgegenstands im Detail offenbart wurde.

Das Mittel (a) enthält mit der oder den organischen Siliciumverbindungen eine Klasse von hochreaktiven Verbindungen, die wie zuvor beschriebenen in Anwesenheit von Wasser eine Hydrolyse bzw. Oligomerisierung und/oder Polymerisieurng eingehen können. Infolge ihrer hohen Reaktivität bilden diese organischen Siliciumverbindungen auf dem Keratinmaterial einen Film aus. Zur Vermeidung der vorzeitigen Oligomerisierung bzw. Polymerisierung kann es für den Anwender von wesentlichem Vorteil sein, das anwendungsbereite Mittel (a) erst kurz vor der Anwendung herstellen.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a1), wobei das Mittel (a1) mindestens eine organische Siliciumverbindung und mindestens ein Tensid enthält, wie sie bei Beschreibung des ersten Erfindungsgegenstands im Detail offenbart wurden, und
- einen zweiten Container mit einem Mittel (a2), wobei das Mittel (a2) Wasser enthält und
- einen dritten Container mit einem Mittel (b), wobei das Mittel (b) mindestens ein Pigment enthält. Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a1`), wobei das Mittel (a1`) mindestens eine organische Siliciumverbindung enthält, wie sie bei Beschreibung des ersten Erfindungsgegenstands im Detail offenbart wurden, und
- einen zweiten Container mit einem Mittel (a2`), wobei das Mittel (a2`) mindestens ein Tensid und Wasser enthält, und
- einen dritten Container mit einem Mittel (b), wobei das Mittel (b) mindestens ein Pigment enthält.

Um eine möglichst lagerstabile Formulierung bereitstellen zu können, wird das Mittel (a1) selbst bevorzugt wasserarm oder wasserfrei konfektioniert.

In einer bevorzugten Ausführungsform ist eine erfindungsgemäße Mehrkomponenten-Verpackungseinheit (Kit-of-parts) dadurch gekennzeichnet, dass das Mittel (a1) - bezogen auf das Gesamtgewicht des Mittels (a1) - einen Wassergehalt von 0,001 bis 10,0 Gew.-%, bevorzugt von 0,5 bis 9,0 Gew.-%, weiter bevorzugt von 1,0 bis 8,0 Gew.-% und ganz besonders bevorzugt von 1,5 bis 7,0 Gew.-% enthält.

Das Mittel (a2) enthält Wasser. In einer bevorzugten Ausführungsform ist eine erfindungsgemäße Mehrkomponenten-Verpackungseinheit (Kit-of-parts) dadurch gekennzeichnet, dass das Mittel (a2) - bezogen auf das Gesamtgewicht des Mittels (a2) - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

Innerhalb dieser Ausführungsform wird das anwendungsbereite Mittel (a) nun durch Vermischen der Mittel (a1) und (a2) hergestellt.

Beispielsweise kann der Anwender das Mittel (a1), welches die organische Siliciumverbindung(en) enthält, zunächst mit dem wasserhaltigen Mittel (a2) verrühren oder verschütteln. Diese Mischung aus (a1) und (a2) kann der Anwender nun - entweder direkt nach ihrer Herstellung oder nach einer kurzen Reaktionszeit von 10 Sekunden bis 20 Minuten - auf die Keratinmaterialien applizieren. Im Anschluss daran kann der Anwender wie zuvor beschrieben das Mittel (b) anwenden.

Betreffend die weiteren bevorzugten Ausführungsformen der erfindungsgemäßen Mehrkomponenten-Verpackungseinheit gilt *mutatis mutantis* das zum erfindungsgemäßen Verfahren gesagte.

### Beispiele

### 1. Formulierungen

Es wurden die folgenden Formulierungen hergestellt:
Vorbehandlungsmittel (a)

| Mittel (a1) | (a11) | (a12) | (a13) | (a14) |
|---|---|---|---|---|
| (3-Aminopropyl)triethoxysilan | 13,3 g | 13,3 g | 13,3 g | 13,3 g |
| Methyltrimethoxysilan | 46,7 g | 46,7 g | 46,7 g | 46,7 g |
| Cetyltrimethylammoniumchlorid | 3,0 g | --- | - | - |
| Quaternium-87 | - | 3,0 g | 1,5 g | - |
| Ceteareth-20 | - | - | 1,5 g | - |
| Wasser | 7,0 g | 7,0 g | 7,0 g | 10,0 g |

| Mittel (a2) | (a21) | (a22) | (a23) | (a24) |
|---|---|---|---|---|
| Cetyltrimethylammoniumchlorid | - | 3,0 g | - | - |
| Quaternium-87 | - | - | 3,0 g | 1,5 g |
| Ceteareth-20 | - | - | --- | 1,5 g |
| Ammoniak/Zitronensäure | ad pH 9,5 | ad pH 9,5 | ad pH 9,5 | ad pH 9,5 |
| Wasser | 100 g | 100 g | 100 g | 100 g |

Färbemittel

| Mittel (b) | (b1) | (b2) |
|---|---|---|
| Colorona Bronze (Merck, Mica, CI77491, Iron oxides, CI77019) | 2,0 | - |
| Colorona Red Gold, Merck, MICA, CI 77891 (TITANIUM DIOXIDE), CI 77491 (IRON OXIDES) | - | 3,0 |
| Dermacryl 79 (Akzo Nobel, Acrylates/Octylacrylamide Copolymer, CAS-Nr. 129702-02-9) | 9,0 | 9,0 |
| Ammoniak (25 %ige wässrige Lösung) | ad pH 10 | ad pH 10 |
| Wasser | ad 100 | ad 100 |

### 2. Anwendung

Zur Herstellung des anwendungsbereiten Vorbehandlungsmittels (a) wurden die jeweils angegebene Menge des gelagerten Mittels (a1) mit der angegebenen Menge des Mittels (a2) unter Schütteln vermischt. Danach wurde das Mittel (a) für 15 Minuten stehen gelassen. Das Mittel (a) ist das anwendungsbereite Mittel.

Jeweils eine Haarsträhne (Kerling, dunkelbraun) wurde in das Mittel (a) getaucht und für 1 Minuten darin belassen. Danach wurde überflüssiges Mittel (a) von jeder Haarsträhne gestreift. Jede Haarsträhne wurde mit Wasser ausgewaschen. Überschüssiges Wasser wurde von jeder Haarsträhne gestreift.

In direktem Anschluss daran wurden die Haarsträhnen jeweils in das Mittel (b) getaucht und für 1 Minute darin belassen. Danach wurde überflüssiges Mittel (b) von jeder Haarsträhne gestreift. Danach wurde jede Haarsträhne gründlich (1 Minute) mit Wasser ausgewaschen und getrocknet.

| Bsp | 1 | 2 | 3 |
|---|---|---|---|
| Mittel (a1) | (a11) | (a12) | (a13) |
| | 5,0 g | 5,0 g | 5,0 g |
| Mittel (a2) | (a21) | (a21) | (a21) |
| | 5,0 g | 5,0 g | 5,0 g |
| Mittel (b) | (b1) | (b1) | (b1) |

| Bsp | 4 | 5 | 6 |
|---|---|---|---|
| Mittel (a1) | (a14) | (a14) | (a14) |
| | 5,0 g | 5,0 g | 5,0 g |
| Mittel (a2) | (a22) | (a23) | (a24) |
| | 5,0 g | 5,0 g | 5,0 g |
| Mittel (b) | (b2) | (b2) | (b2) |

Bei den Beispielen 1 bis 6 wurden sehr intensive und sehr gleichmäßige Färbungen erhalten. Im Fall der Beispiele 4, 5, und 6 wurde eine rotgolden schimmernde Strähne erhalten, die im Vergleich zum eingesetzten dunkelbraunen Haar aufgehellt war.

## Patentansprüche

1. Verfahren zum Färben von keratinischem Material, insbesondere menschlichen Haaren, umfassend die folgenden Schritte:
- Anwendung eines Mittels (a) auf dem keratinischem Material, wobei das Mittel (a) mindestens eine organische Siliciumverbindung der Formel (I) und mindestens eine organische Silciumverbindung der Formel (IV) und mindestens ein Tensid enthält,
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe steht,
- R₃, R₄ unabhängig voneinander für eine Methylgruppe oder für eine Ethylgruppe stehen,
- a für die Zahl 3 steht und
- b für die Zahl 0 steht,
R₉Si(OR₁₀)ₖ(R₁₁)ₘ (IV),
wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
- Anwendung eines Mittels (b) auf dem keratinischen Material, wobei das Mittel (b) mindestens ein Pigment enthält.

2. Verfahren nach Anspruch 1, **dadurch gekennzeichnet, dass** das Mittel (a) mindestens eine organische Silicumverbindung der Formel (I) enthält,
R₁R₂-N-L-Si(OR₃)ₐ(R₄)_{b} (I),
wobei
- L für eine Propylengruppe (-CH₂-CH₂-CH₂-) oder für eine Ethylengruppe (-CH₂-CH₂-), steht,

3. Verfahren nach einem der Ansprüche 1 bis -3- 2, **dadurch gekennzeichnet, dass** das Mittel (a) mindestens eine organische Siliciumverbindung enthält, die ausgewählt ist aus der Gruppe aus
- (3-Aminopropyl)trimethoxysilan
- (3-Aminopropyl)triethoxysilan
- (2-Aminoethyl)trimethoxysilan und
- (2-Aminoethyl)triethoxysilan

4. Verfahren nach einem der Ansprüche 1 bis 3, **dadurch gekennzeichnet, dass** das Mittel (a) mindestens eine organische Siliciumverbindung enthält, die ausgewählt ist aus der Gruppe aus
- Methyltrimethoxysilan
- Methyltriethoxysilan
- Ethyltrimethoxysilan
- Ethyltriethoxysilan
- Octyltrimethoxysilan
- Octyltriethoxysilan
- Dodecyltrimethoxysilan und
- Dodecyltriethoxysilan.

5. Verfahren nach einem der Ansprüche 1 bis 4, **dadurch gekennzeichnet, dass** das Mittel (a) - bezogen auf das Gesamtgewicht des Mittels (a) - enthält:
- 0,5 bis 3,0 Gew.-% mindestens einer ersten organischen Silicumverbindung, die ausgewählt ist aus der Gruppe aus (3-Aminopropyl)trimethoxysilan, (3-Aminopropyl)triethoxysilan, (2-Aminoethyl)tri-methoxysilan und (2-Aminoethyl)triethoxysilan, und
- 3,2 bis 10,0 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Octyltrimethoxysilan, Octyltriethoxysilan, Dodecyltrimethoxysilan und Dodecyltriethoxysilan.

6. Verfahren nach einem der Ansprüche 1 bis 5, **dadurch gekennzeichnet, dass** das Mittel (a) mindestens ein Tensid aus der Gruppe der nichtionischen und/oder kationischen Tenside enthält.

7. Verfahren nach einem der Ansprüche 1 bis 6, **dadurch gekennzeichnet, dass** das Mittel (a) mindestens ein kationisches Tensid der Formel (X) enthält, 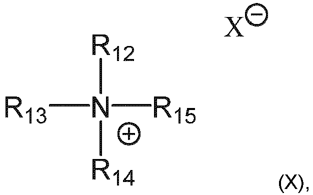 worin
R12, R13, R14 unabhängig voneinander für eine C₁-C₆-Alkylgruppe, eine C₂-C₆-Alkenylgruppe oder eine C₂-C₆-Hydroxyalkylgruppe stehen,
R15 für eine C₈-C₂₈-Alkylgruppe steht und
X- für ein physiologisch verträgliches Anion steht,

8. Verfahren nach einem der Ansprüche 1 bis 7, **dadurch gekennzeichnet, dass** das Mittel (a) mindestens ein kationisches Tensid der Formel (XI) enthält, 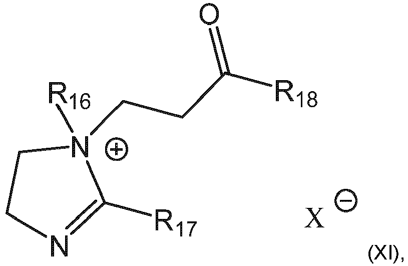 worin
R16 für eine C₁-C₆-Alkylgruppe steht
R17, R18 unabhängig voneinander für eine C₇-C₂₇-Alkylgruppe stehen und
X- für ein physiologisch verträgliches Anion steht.

9. Verfahren nach einem der Ansprüche 1 bis 8, **dadurch gekennzeichnet, dass** das Mittel (a) mindestens ein kationisches Tensid de Formel (XII) enthält, 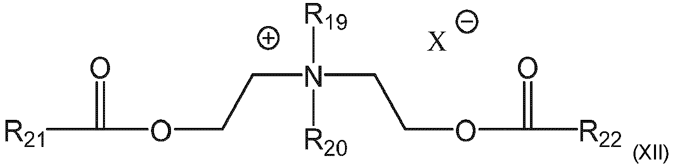 worin
R19, R20 unabhängig voneinander für eine C₁-C₆-Alkylgruppe oder eine C₂-C₆-Hydroxyalkylgruppe stehen,
R21, R22 unabhängig voneinander für eine C₇-C₂₇-Alkylgruppe stehen und
X- für ein physiologisch verträgliches Anion steht.

10. Verfahren nach einem der Ansprüche 1 bis 44 9, **dadurch gekennzeichnet, dass** das Mittel (b) mindestens ein anorganisches Pigment enthält, das ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

11. Verfahren nach einem der Ansprüche 1 bis 10, **dadurch gekennzeichnet, dass** das Mittel (b) mindestens ein organisches Pigment enthält, das ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

12. Verfahren nach einem der Ansprüche 1 bis 11, **dadurch gekennzeichnet, dass** das Mittel (b) mindestens ein filmbildendes, hydrophobes Polymer enthält.

13. Verfahren nach einem der Ansprüche 1 bis 4& 12, **dadurch gekennzeichnet**, das zunächst das Mittel (a) angewendet wird, danach das Mittel (b) angewendet wird, wobei der Zeitraum zwischen der Anwendung der Mittel (a) und (b) bei maximal 24 Stunden, bevorzugt bei maximal 12 Stunden, weiter bevorzugt bei maximal 6 Stunden und ganz besonders bevorzugt bei maximal 30 Minuten liegt.

14. Verfahren nach einem der Ansprüche 1 bis 46 13, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

15. Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung der Formel (I) und mindestens eine organische Silciumverbindung der Formel (IV) und mindestens ein Tensid enthält, wie sie in den Ansprüchen 1 bis 9 beschrieben sind, und
- einen zweiten Container mit einem Mittel (b), wobei das Mittel (b) mindestens ein Pigment enthält, wie es in den Ansprüchen 1, 10, 44 11 und/oder 4& 12 beschrieben ist.

## Claims

1. Process for coloring keratinous material, in particular human hair, comprising the following steps:
- Application of an agent (a) to the keratinous material, wherein the agent (a) comprises at least one organic silicon compound of formula (I) and at least one organic silicon compound of formula (IV) and at least one surfactant,
R₁R₂N-L-Si(OR₃)ₐ(R4)_{b} (I),
whereby
- R₁, R₂ both represent a hydrogen atom, and
- L stands for a linear, divalent C₁-C₆-alkylene group,
- R₃, R₄ independently of one another represent a methyl group or an ethyl group,
- a stands for the number 3 and
- b stands for the number 0,
R₉Si(OR₁₀)ₖ(R₁₁)ₘ (IV),
whereby
- R₉ represents a C₁ -C₁₂ alkyl group,
- R₁₀ represents a hydrogen atom or a C₁-C₆ alkyl group,
- R₁₁ stands for a C₁-C₆ alkyl group
- k is an integer from 1 to 3, and
- m stands for the integer 3 - k, and
- application of an agent (b) to the keratinous material, the agent (b) containing at least one pigment.

2. Process according to claim 1, **characterized in that** the agent (a) contains at least one organic silicon compound of the formula (I),
R₁R₂N-L-Si(OR₃)ₐ(R₄)_{b} (I),
whereby
- L stands for a propylene group (-CH₂-CH₂-CH₂-) or for an ethylene group (-CH₂-CH₂-).

3. Process according to one of claims 1 to 2, **characterized in that** the agent (a) contains at least one organic silicon compound selected from the group consisting of
- (3-aminopropyl)trimethoxysilane
- (3-aminopropyl)triethoxysilane
- (2-aminoethyl)trimethoxysilane and
- (2-aminoethyl)triethoxysilane

4. Process according to any one of claims 1 to 3, **characterized in that** the agent (a) contains at least one organic silicon compound selected from the group consisting of
- Methyltrimethoxysilane
- Methyltriethoxysilane
- Ethyltrimethoxysilane
- Ethyltriethoxysilane
- Octyltrimethoxysilane
- Octyltriethoxysilane
- Dodecyltrimethoxysilane and
- Dodecyltriethoxysilane.

5. Process according to one of claims 1 to 4, **characterized in that** the agent (a) contains - based on the total weight of the agent (a):
- from 0.5 to 3.0% by weight of at least one first organic silica compound selected from the group consisting of (3-aminopropyl)trimethoxysilane, (3-aminopropyl)triethoxysilane, (2-aminoethyl)trimethoxysilane and (2-aminoethyl)triethoxysilane, and
- 3.2 to 10.0% by weight of at least one second organic silicon compound selected from the group consisting of methyltrimethoxysilane, methyltriethoxysilane, ethyltrimethoxysilane, ethyltriethoxysilane, octyltrimethoxysilane, octyltriethoxysilane, dodecyltrimethoxysilane and dodecyltriethoxysilane.

6. Process according to one of claims 1 to 5, **characterized in that** the agent (a) contains at least one surfactant from the group of non-ionic and/or cationic surfactants.

7. Process according to one of claims 1 to 6, **characterized in that** the agent (a) contains at least one cationic surfactant of the formula (X), 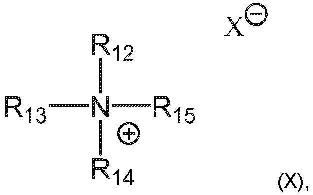 wherein
R12, R13, R14 independently of one another represent a C₁-C₆-alkyl group, a C₂-C₆-alkenyl group or a C₂-C₆-hydroxyalkyl group,
R15 represents a C₈-C₂₈ alkyl group and
X- stands for a physiologically compatible anion.

8. Process according to one of claims 1 to 7, **characterized in that** the agent (a) contains at least one cationic surfactant of the formula (XI), 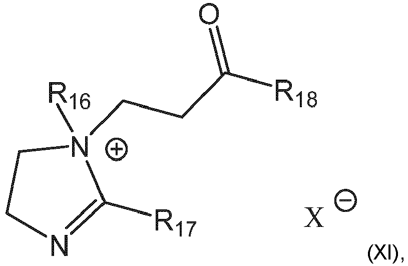 wherein
R16 represents a C₁-C₆ alkyl group
R17, R18 independently of one another represent a C₇-C₂₇ alkyl group and
X- stands for a physiologically compatible anion.

9. Process according to one of claims 1 to 8, **characterized in that** the agent (a) contains at least one cationic surfactant of formula (XII), 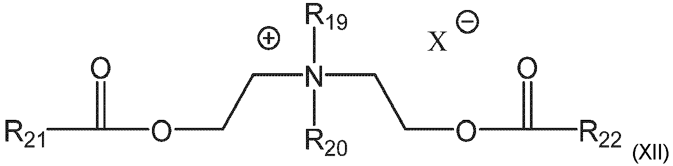 wherein
R19, R20 independently of one another represent a C₁-C₆ alkyl group or a C₂-C₆ hydroxyalkyl group,
R21, R22 independently of one another represent a C₇-C₂₇ alkyl group and
X- stands for a physiologically compatible anion.

10. Process according to any one of claims 1 to 9, **characterized in that** the agent (b) at least one inorganic pigment selected from the group consisting of colored metal oxides, metal hydroxides, metal oxide hydrates, silicates, metal sulfides, complex metal cyanides, metal sulfates, bronze pigments and/or colored pigments based on mica or mica coated with at least one metal oxide and/or one metal oxychloride.

11. Process according to any one of claims 1 to 10, **characterized in that** the agent (b) contains at least one organic pigment selected from the group consisting of carmine, quinacridone, phthalocyanine, sorgho, blue pigments with the Color Index Numbers CI 42090, CI 69800, CI 69825, CI 73000, CI 74100, CI 74160, yellow pigments with the Color Index Numbers CI 11680, CI 11710, CI 15985, CI 19140, CI 20040, CI 21100, CI 21108, CI 47000, CI 47005, green pigments with the color index numbers CI 61565, CI 61570, CI 74260, orange pigments with the color index numbers CI 11725, CI 15510, CI 45370, CI 71105, red pigments with the color index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

12. Process according to one of claims 1 to 11, **characterized in that** the agent (b) contains at least one film-forming, hydrophobic polymer.

13. Process according to one of claims 1 to 12, **characterized in that** first the agent (a) is applied, then the agent (b) is applied, the period between the application of the agents (a) and (b) being at most 24 hours, preferably at most 12 hours, further preferably at most 6 hours and very particularly preferably at most 30 minutes.

14. Process according to any one of claims 1 to 13, comprising the following steps in the order given
(1) Application of the product (a) on the keratinous material,
(2) Allow the agent (a) to act for a period of 10 seconds to 10 minutes, preferably from 10 seconds to 5 minutes,
(3) If necessary, rinse the keratinous material with water,
(4) Application of agent (b) on the keratinous material,
(5) allowing the agent (b) to act for a period of 30 seconds to 30 minutes, preferably from 30 seconds to 10 minutes, and
(6) Rinse the keratinous material with water.

15. Multi-component packaging unit (kit-of-parts) for coloring keratinous material, comprising separately assembled
- a first container with an agent (a), wherein the agent (a) contains at least one organic silicon compound of formula (I) and at least one organic silicon compound of formula (IV) and at least one surfactant as described in claims 1 to 9, and
- a second container with an agent (b), wherein the agent (b) contains at least one pigment as described in claims 1, 10, 11 and/or 12.

## Revendications

1. Procédé de coloration de matière kératinique, en particulier de cheveux humains, comprenant les étapes suivantes :
- Application d'un agent (a) sur la matière kératinique, l'agent (a) contenant au moins un composé organique du silicium de formule (I) et au moins un composé organique du silicium de formule (IV) et au moins un agent tensioactif,
R₁R₂N-L-Si(OR₃)ₐ(R₄)_{b} (I),
où
- R₁ , R₂ représentent tous deux un atome d'hydrogène, et
- L représente un groupe alkylène linéaire divalent Ci-Ce
- R₃, R₄ représentent indépendamment l'un de l'autre un groupe méthyle ou un groupe éthyle,
- a représente le nombre 3 et
- b représente le nombre 0,
R₉Si(OR₁₀)ₖ(R₁₁)ₘ (IV),
où
- R₉ représente un groupe alkyle en C₁ -C₁₂
- R₁₀ représente un atome d'hydrogène ou un groupe alkyle en C₁ -C₆
- R₁₁ représente un groupe alkyle en C₁ -C₆
- k représente un nombre entier de 1 à 3, et
- m représente le nombre entier 3 - k, et
- l'application d'un agent (b) sur la matière kératinique, ledit agent (b) contenant au moins un pigment.

2. Procédé selon la revendication 1, **caractérisé en ce que** l'agent (a) contient au moins un composé de silicium organique de formule (I),
R₁R₂N-L-Si(OR₃)ₐ(R₄)_{b} (I),
où
- L représente un groupe propylène (-CH₂-CH₂-CH₂-) ou un groupe éthylène (-CH₂-CH₂-).

3. Procédé selon l'une quelconque des revendications 1 à 2, **caractérisé en ce que** l'agent (a) contient au moins un composé organique du silicium choisi dans le groupe constitué par
- (3-aminopropyl)triméthoxysilane
- (3-aminopropyl)triéthoxysilane
- (2-aminoéthyl)triméthoxysilane et
- (2-aminoéthyl)triéthoxysilane

4. Procédé selon l'une quelconque des revendications 1 à 3, **caractérisé en ce que** l'agent (a) contient au moins un composé organique du silicium choisi dans le groupe constitué par
- Méthyltriméthoxysilane
- Méthyltriéthoxysilane
- Éthyltriméthoxysilane
- Éthyltriéthoxysilane
- Octyltriméthoxysilane
- Octyltriéthoxysilane
- dodécyltriméthoxysilane et
- Dodécyltriéthoxysilane.

5. Procédé selon l'une des revendications 1 à 4, **caractérisé en ce que** l'agent (a) contient - par rapport au poids total de l'agent (a) :
- 0,5 à 3,0 % en poids d'au moins un premier composé de silicium organique choisi dans le groupe constitué par le (3-aminopropyl)triméthoxysilane, le (3-aminopropyl)triéthoxysilane, le (2-aminoéthyl)triméthoxysilane et le (2-aminoéthyl)triéthoxysilane, et
- 3,2 à 10,0 % en poids d'au moins un second composé organique du silicium choisi dans le groupe constitué par le méthyltriméthoxysilane, le méthyltriéthoxysilane, l'éthyltriméthoxysilane, l'éthyltriéthoxysilane, l'octyltriméthoxysilane, l'octyltriéthoxysilane, le dodécyltriméthoxysilane et le dodécyltriéthoxysilane.

6. Procédé selon l'une des revendications 1 à 5, **caractérisé en ce que** l'agent (a) contient au moins un tensioactif du groupe des tensioactifs non ioniques et/ou cationiques.

7. Procédé selon l'une quelconque des revendications 1 à 6, **caractérisé en ce que** l'agent (a) contient au moins un agent tensioactif cationique de formule (X), 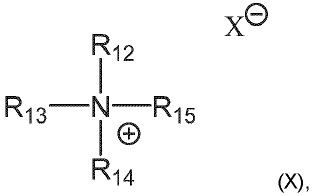 où
R12, R13, R14représentent, indépendamment les uns des autres, un groupe alkyle en Ci-Ce, un groupe alcényle en C₂-C₆ ou un groupe hydroxyalkyle en C₂-C₆,
R15 représente un groupe alkyle en C₈-C₂₈ et
X- représente un anion physiologiquement acceptable.

8. Procédé selon l'une quelconque des revendications 1 à 7, **caractérisé en ce que** l'agent (a) contient au moins un agent tensioactif cationique de formule (XI), 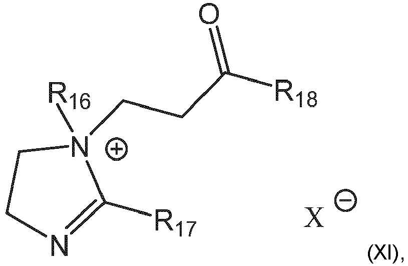 où
R16 représente un groupe alkyle en C₁ -C₆
R17, R18 représentent indépendamment un groupe alkyle en C₇-C₂₇ et
X- représente un anion physiologiquement acceptable.

9. Procédé selon l'une des revendications 1 à 8, **caractérisé en ce que** l'agent (a) contient au moins un agent tensioactif cationique de formule (XII), 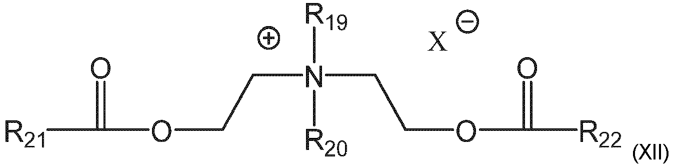 où
R19, R20 représentent indépendamment l'un de l'autre un groupe alkyle en Ci-Ce ou un groupe C₂-C₆-hydroxyalkyle,
R21, R22 représentent indépendamment un groupe C₇ -C₂₇-alkyle et
X- représente un anion physiologiquement acceptable.

10. Procédé selon l'une quelconque des revendications 1 à 9, **caractérisé en ce que** l'agent (b) contient au moins un pigment inorganique qui est choisi dans le groupe des oxydes métalliques colorés, des hydroxydes métalliques, des oxydes métalliques hydratés, des silicates, des sulfures métalliques, des cyanures métalliques complexes, des sulfates métalliques, des pigments de bronze et/ou des pigments colorés à base de mica ou de mica, qui sont revêtus d'au moins un oxyde métallique et/ou d'un oxychlorure métallique.

11. Procédé selon l'une des revendications 1 à 10, **caractérisé en ce que** la composition (b) contient au moins un pigment organique choisi dans le groupe constitué par le carmin, la quinacridone, la phtalocyanine, le sorgho, les pigments bleus ayant les numéros d'indice de couleur CI 42090, CI 69800, CI 69825, CI 73000, CI 74100, CI 74160, les pigments jaunes ayant les numéros d'indice de couleur CI 11680, CI 11710, CI 15985, CI 19140, CI 20040, CI 21100, CI 21108, CI 47000, CI 47005, pigments verts avec les numéros Color Index CI 61565, CI 61570, CI 74260, pigments orange avec les numéros Color Index CI 11725, CI 15510, CI 45370, CI 71105, pigments rouges avec les numéros Color Index CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

12. Procédé selon l'une des revendications 1 à 11, **caractérisé en ce que** l'agent (b) contient au moins un polymère hydrophobe filmogène.

13. Procédé selon l'une des revendications 1 à 12, **caractérisé en ce que** l'on applique d'abord l'agent (a), puis l'agent (b), l'intervalle entre l'application des agents (a) et (b) étant de 24 heures au maximum, de préférence de 12 heures au maximum, de préférence encore de 6 heures au maximum et tout particulièrement de 30 minutes au maximum.

14. Procédé selon l'une quelconque des revendications 1 à 13, comprenant les étapes suivantes dans l'ordre indiqué
(1) Application de l'agent (a) sur la matière kératinique,
(2) laisser agir l'agent (a) pendant une période de 10 secondes à 10 minutes, de préférence de 10 secondes à 5 minutes,
(3) le cas échéant, rinçage à l'eau de la matière kératinique,
(4) Application de l'agent (b) sur la matière kératinique,
(5) laisser agir l'agent (b) pendant une durée de 30 secondes à 30 minutes, de préférence de 30 secondes à 10 minutes, et
(6) Rincer la matière kératinique à l'eau.

15. Unité d'emballage à plusieurs composants (Kit-of-parts) pour la coloration de matières kératiniques, comprenant, confectionnés séparément les uns des autres
- un premier conteneur comprenant un agent (a), ledit agent (a) contenant au moins un composé organique du silicium de formule (I) et au moins un composé organique du silicium de formule (IV) et au moins un agent tensioactif, tels que décrits dans les revendications 1 à 9, et
- un deuxième conteneur avec un agent (b), l'agent (b) contenant au moins un pigment tel que décrit dans les revendications 1, 10, 11 et/ou 12.
